# EUROPEAN PATENT SPECIFICATION

(11) **EP 1 901 717 B1**
(45) Date of publication and mention of the grant of the patent: **18.07.2012**
(21) Application number: 06780772.7
(22) Date of filing: 28.06.2006
(51) Int. Cl.: A61K 31/4709, A61K 9/16

(54) **ORALLY DISINTEGRATING POWDER COMPRISING CILOSTAZOL AND MANNITOL**
SICH IM MUND AUFLÖSENDES PULVER MIT CILOSTAZOL UND MANNITOL
POUDRE A DESINTEGRATION PAR VOIE ORALE, COMPRENANT DU CILOSTAZOL ET DU MANNITOL

(30) Priority: 29.06.2005 JP 2005190156
(43) Date of publication of application: 26.03.2008
(73) Proprietor: OTSUKA PHARMACEUTICAL CO., LTD., Chiyoda-ku Tokyo 101-8535 (JP)
(72) Inventor: TODA, Masafumi Otsuka Pharmaceutical Co., Ltd., Tokushima-shi, Tokushima 771-0182 (JP); MUKAI, Tadashi Otsuka Pharmaceutical Co., Ltd., Tokushima-shi, Tokushima 771-0182 (JP)
(74) Representative: von Kreisler Selting Werner
(86) International application number: PCT/JP2006/313345
(87) International publication number: WO 2007/001086

(56) References cited:
- EP-A- 1 475 084
- EP-A- 1 488 811
- WO-A-00/57881
- WO-A2-01/39749

## Description

### TECHNICAL FIELD

The present invention relates to oral powder comprising cilostazol which can be disintegrated in oral cavity.

### BACKGROUND ART

Cilostazol is 6-[4-(1-cyclohexyl-1H-tetrazol-5-yl)-butoxy]-3,4-dihydrocarbostyril as shown in the following formula (1), which exhibits high inhibitory action for platelet aggregation as well as inhibitory action for phosphodiesterase, antitumor activity, hypotensive action, antiphlogistic action, etc. and thereby is widely used as an antithrombotic agent, a drug improving cerebral circulation, an antiphlogistics, an antitumor drug, an antihypertensive agent, an antiasthmatic agent, as well as a phosphodiesterase inhibitor. The cilostazol tablets which are called Pletaal tablet 50^{®} and Pletaal tablet 100^{®} (OTSUKA PHARMACEUTICAL CO., LTD.) have already been on sale (see JP-A-56-49378). Further, the tablets have been additionally approved as a medicament having an indication which prevents the relapse after treatment of cerebral infarction (except cardiogenic cerebral infarction).

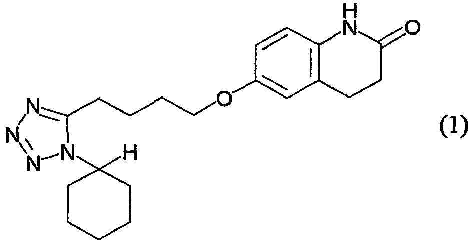

The use state of Pletaal tablet^{®} was investigated (market research between January and June, 2001), and therein the distribution classified by age bracket had shown that patients of 65 years or more occupied about 74% of the whole. According to the distribution classified by age bracket in connection with cerebral infarction-patients that has been newly applied since 2003, patients of 65 years or more occupied about 83.6% (Table 1). Anyway, it appears that many of patients to whom the cilostazol tablets are administered are aged people.

**Table 1. The number of cerebral infarction-patients classified by age bracket.**

| Age | The number of cerebral infarction-patients |
|---|---|
| less than 65 years old | 174 thousand (16.4 %) |
| 65 years to 75 years old | 334 thousand (31.4 %) |
| 75 years to 85 years old | 367 thousand (34.5%) |
| more than 85 years old | 189 thousand (17.8 %) |
| Total | 1064 thousand |

Generally, as a person gets older, his eating/swallowing function comes to be lowered. Accordingly, it has been desired to develop a drug preparation which is easy to be taken by aged patients to whom the cilostazol tablet is applied.

Furthermore, it is known that there are some patients suffering from dysphagia among cerebral infarction-patients to whom the cilostazol tablet is applied. When administering a drug to such patients, the drug is milled and given via percutaneous endoscopic gastrostomy or tube feeding in case of severe disorder (e.g. aspiration of salivary, aspiration of food). And in case of minor to medium disorder (aspiration of water, occasional aspiration), actually the drug is orally given with some ideas, for example, given together with jelly, pudding, or rice porridge, etc., or administered using porridgy liquid instead of water. It is known that patients suffering from aspiration of water or occasional aspiration can swallow salivary though it is difficult for the patients to drink water.

In the Silver Science Research which has been sponsored by the Japanese Ministry of Health and Welfare since 1988, Sugihara et al. who worked at a section "study for producing a new drug preparation and a new package suitable for administering to aged people" investigated with questionnaire what kind of a drug formulation aged people hoped would be developed in the future. In result, it was reported that many patients hoped semisolid formulations such as jelly, yogurt, pudding, etc.

In the Research, furthermore, Sugihara et al. has been researching orally solving formulations and pasty formulations which are possible to be orally administered without water in order that aged patients can be given a drug more safely and surely. In their research, they investigated the difficulty to take a drug preparation or other investigation items by administering an orally solying formulation not including a medicament to 128 example subjects (average of their ages: 77.4±8.5), and administering a pasty formulation not including a medicament to 73 example subjects (average of their ages: 78.4±9.1). In result, the answers "easy to take" and "easy to administer" were 82.2% and 75.8%, respectively in case of the orally solving formulation; 60.6%, 72.1%, respectively in case of the pasty formulation. In this way, it is concluded that both of the drug formulations are easy to be taken for aged people.

WO 00/57881 discloses a cilostazol powder containing 28% mannitol, SCS and hydroxypropyl cellulose or polyarylalcohol acting as disintegrator. Further, mannitol containing tablets or granules are known from WO 01(39749, EP-A-1475084 and EP-A-1488811.

Under the situation that such various formulations are needed, cilostazol has been also studied about various formulations (see JP-A-2001-163769), furthermore, it has been desired to develop a drug formulation which is easy to be taken by many patients to whom cilostazol tablets are applied, especially aged patients and patients suffering from dysphagia in order to get the medicament used suitably.

### DISCLOSURE OF INVENTION

### (Problem to be solved by the invention)

In compliance with above-mentioned needs, the inventors tried to produce tablets which comprise 10% by weight to 30% by weight, i.e. 50 mg to 100 mg of cilostazol as a single dosage unit and which is an orally disintegrating tablet widely used in the art. However, though tablets comprising cilostazol were produced as an orally disintegrating formulation, the tablets were difficult to disintegrate because of the water-insolubility of cilostazol, even using sugar or sugar alcohol which is generally used in orally disintegrating formulation and can promote rapid dissolution. Alternatively, in order to overcome the water-insolubility, a ratio of the solubilizer was increased and a ratio of cilostazol was decreased. However, it came to be necessary to produce a big tablet since the single dosage unit of cilostazol is high. Accordingly, a tablet having a maximum tablet size which can be prepared in general, such as 12 mm of diameter exhibited slow disintegration rate, hard disintegrability in oral cavity, a sandy feeling and a bad feeling in oral cavity when administered.

In addition, fine powders comprising cilostazol were produced in consideration of a feeling in oral cavity, however, they could not be taken without water because of a sandy or dry feeling thereof in oral cavity.

Therefore, it has been desired to develop a new cilostazol formulation which is different from an orally disintegrating tablet and the like and is possible to be taken without water, so that is possible to be easily taken by aged patients whose swallowing function is lowered or patients suffering from dysphagia as a sequela of cerebral infarction.

### (Means to solve the problem)

The present inventors have extensively studied a variety of formulations of cilostazol preparations to reach for the above object, and have found that a powder formulation thereof wherein mannitol is formulated could be an orally disintegrable formulation. In the intrabuccal disintegration of the powder, the excipients are disintegrated in oral cavity and then the cilostazol powder becomes to be in a dispersed state. The particle size of the cilostazol is about 20 µm, and the particle is agreeable in oral cavity and easy to be swallowed. In addition, other sugars or sugar alcohols except mannitol were not suitable since they are too sweet or high humidity. Furthermore, the present inventors have extensively studied and found that a powder comprising cilostazol which further comprises 70% by weight or more mannitol has a fully fast disintegrating speed and a fully good feeling in oral cavity. The present invention has been completed based on these findings. As far as the present inventors know, there is no pharmaceutical powder designed to get it disintegrated in oral cavity and get it taken without water until now. Even if such powder exists, it would be thought that it is minor.

An object of the present invention is to provide an orally disintegrating powder comprising cilostazol that can be disintegrated in oral cavity and can be taken without water by a lot of patients to whom cilostazol is applied, especially aged patients and patients suffering from dysphagia.

The present invention provides an orally disintegrating powder comprising cilostazol as an active ingredient which further comprises mannitol in an amount of 70% by weight or more, which can be disintegrated in oral cavity and can be taken without water.

In addition, the present invention provides the above-mentioned orally disintegrating powder comprising cilostazol wherein an amount of the cilostazol is 10% by weight to 30% by weight.

Furthermore, the present invention provides the above-mentioned orally disintegrating powder comprising cilostazol wherein a single dosage unit of the cilostazol is 50 mg to 100 mg.

The preferable mannitol is D-mannitol.

The feeling of the powder in oral cavity is especially preferable when using D-mannitol derived from corn starch, i.e. D-mannitol whose starting material is corn starch.

Furthermore, an orally disintegrating powder comprising cilostazol wherein a mean particle size of the above-mentioned mannitol is 20 micrometers to 80 micrometers is suitable.

An embodiment of the present invention includes the above-mentioned orally disintegrating powder comprising cilostazol which further comprises 5% by weight or less microcrystalline cellulose.

The above-mentioned powder may optionally comprise an ingredient(s) for pharmaceutical preparation which can be generally formulated in a pharmaceutical preparation, and the examples of the ingredient include excipients, binders, lubricants, disintegrating agents, colorants, flavors, sweeteners, glidants, stabilizers, etc.

An embodiment of the present invention includes the use of the above-mentioned orally disintegrating powder comprising cilostazol and mannitol for preparing a medicament for preventing relapse of cerebral infarction. Cilostazol can be prepared according to the method set forth in, for example, JP-A-56-49378.

The mannitol used herein may include, but are not limited to, D-mannitol: PEARLITOL 50C^{®} (manufactured by ROQUETTE); D-mannitol: Mannit Kyowa^{®} (manufactured by KYOWA HAKKO KOGYO Co.,Ltd.); etc.

The microcrystalline cellulose used herein may include, but is not limited to, Ceolus PH301 (manufactured by AsahiKASEI), etc.

The term "orally disintegrating powder" used herein means a powder wherein excipients are disintegrated in oral cavity and then the cilostazol powder becomes to be in a dispersed state, which is agreeable in oral cavity and easy to be swallowed.

### (Effect of the invention)

According to the above-mentioned embodiments, the present invention provides a new cilostazol formulation that can be disintegrated in oral cavity and can be taken without water by a lot of patients to whom cilostazol has been applied, especially aged patients and patients suffering from dysphagia.

In the orally disintegrating powder comprising cilostazol of the present invention, 70% by weight or more D-mannitol is formulated, and it is suitable that the particle size of the D-mannitol is 20 micrometers to 80 micrometers. Furthermore the feeling of the powder in oral cavity was especially preferable when D-mannitol whose starting material was corn starch was used and the formulated amount of microcrystalline cellulose was 5% by weight or less.

Hereinafter, the orally disintegrating powder of the present invention is explained showing Examples, and the experimental results of the feeling in oral cavity using the above powder is also explained compared with the feeling of a corresponding orally disintegrating tablet and ordinary powder.

### Example 1

795 g of D-mannitol (PEARLITOL 50C, manufactured by ROQUETTE) and 200 g of cilostazol powder were charged in a vertical granulator (VG-10, produced by Powrex corp.) and then mixed. Thereto 125 g of 4% by weight hydroxypropylcellulose (HPC-L, manufactured by NIPPON SODA CO., LTD.) was added and the mixture was granulated. The produced granules were dried at 70°C in a tray type dryer and then sifted by means of sieve of 500 micrometers opening to give a powder containing 20% by weight cilostazol.

### Example 2

In similar manner as described in Example 1, using 770 g of D-mannitol (PEARLITOL 50C, manufactured by ROQUETTE), 25 g of microcrystalline cellulose (Ceolus PH301, manufactured by AsahiKASEI) and 200 g of cilostazol powder, a powder containing 20 % by weight cilostazol was prepared.

### Example 3

766 g of D-mannitol (PEARLITOL 50C, manufactured by ROQUETTE), 25 g of microcrystalline cellulose (Ceolus PH301, manufactured by AsahiKASEI) and 200 g of cilostazol powder were charged in a vertical granulator (VG-10, produced by Powrex corp.) and then mixed. Thereto 125 g of 4 % by weight hydroxypropylcellulose (HPC-L, manufactured by NIPPON SODA CO., LTD.) was added and the mixture was granulated. The produced granules were dried at 70°C in a tray type dryer and then sifted by means of sieve of 500 micrometers. Thereto 5 g of light anhydrous silicic acid (ADSOLIDER 101, manufactured by Freund Industrial Co., Ltd.) as a glidant was added, and the mixture was mixed to give a powder containing 20% by weight cilostazol.

### Reference Example 1

192 g of erythritol (manufactured by Nikken Chemicals Co., Ltd.) and 100 g of corn starch (Nisshoku corn starch, manufactured by NIHON SHOKUHIN KAKO CO. LTD.), 8 g of hydroxypropylcellulose (HPC-L, manufactured by NIPPON SODA CO., LTD.) and 100 g of cilostazol powder were charged in a fluid bed granulating/drying apparatus Multiplex (MP-01, produced by Powrex corp.). The mixture was granulated during spraying purified water as a binder and the resulting granules are directly dried to give Granule A. Per 400 g of Granule A, 40 g of PVP-XL (manufactured by ISP) as a disintegrating agent and 2 g of magnesium stearate as a lubricant were added to the Granule A. And the mixture was compressed by means of a continuous tabletting machine 812HUK (made by Kikusui Seisakusho Ltd.) to give tablets containing 100 mg of cilostazol (weighing 442 mg per a tablet and having 12 mm in diameter).

### Reference Example 2

94 g of corn starch (Nisshoku corn starch, manufactured by-NIHON SHOKUHIN KAKO CO. LTD.), 6 g of hydroxypropylcellulose (HPC-L, manufactured by NIPPON SODA CO., LTD.) and 100 g of cilostazol powder were charged in a fluid bed granulating/drying apparatus Multiplex (MP-01, produced by Powrex corp.). The mixture was granulated during spraying purified water as a binder and the resulting granules are directly dried to give Granule B. Per the Granule B, 0.5% by weight magnesium stearate as a lubricant was added to the Granule B. And the mixture was compressed by means of a continuous tabletting machine 812HUK (made by Kikusui Seisakusho Ltd.) to give tablets containing 100 mg of cilostazol (weighing 201 mg per a tablet and having 9 mm in diameter).

### Reference Example 3

560 g of lactose (manufactured by H.M.S.), 300 g of corn starch (Nisshoku corn starch, manufactured by NIHON SHOKUHIN KAKO CO. LTD.) and 100 g of cilostazol powder were charged in a fluid bed granulating/drying apparatus Multiplex (MP-01, produced by Powrex corp.). The mixture was granulated during spraying 3% by weight hydroxypropylcellulose (HPC-L, manufactured by NIPPON SODA CO., LTD.) as a binder (the sprayed amount of hydroxypropylcellulose as a solid was 30 g). The resulting granules are directly dried to give Granule C. The Granule C was sifted by means of sieve of 500 µm opening and thereto 10 g of light anhydrous silicic acid (Aerosil, Nippon Aerosil) was added and mixed to give a powder containing 10% by weight cilostazol.

### Experiment 1

Each of the preparations of Examples 1 to 3 and Reference Examples 1 to 3 wherein each includes 100 mg of cilostazol was thrown into a mouth of a subject and then disintegrated on his tongue. And the disintegration time obtained from each trial was compared each other. In the case of the powders of Examples 1 to 3 and Reference Example 3, the disintegration time was defined as the time taken until the subject came to feel agreeable, and the comparison was carried out with these times.

**Table 2**

| Sample | Example 1 | Example 2 | Example 3 | Reference Example 1 | Reference Example 2 | Reference Example 3 |
|---|---|---|---|---|---|---|
| Disintegration time | Not more than 15 sec. | Not more than 15 sec. | Not more than 15 sec. | Not less than 1 min. | Not less than 3 min. | Not less than 3 min. |
| Feeling in oral cavity | agreeable | agreeable | almost agreeable | sandy, unagreeable | sandy, unagreeable | sandy, unagreeable |

As shown in Table 2, with regard to the powders of Examples 1 to 3, each time taken until the subject came to feel agreeable was short, and each feeling in oral cavity was good. On the other hand, with regard to the tablets of Reference Examples 1 and 2 and the powder of Example 3, each disintegration time was long, and each unagreeable feeling in oral cavity continued even after disintegration.

### Experiment 2

Each 500 mg of the powders of Examples 1 and 2 or 1000 mg of the powder of Reference Example 3 was placed in a mouth of a subject and then disintegrated on his tongue. And the feeling in oral cavity obtained from each trial was compared each other. The evaluation of the feeling in oral cavity was carried out with 10 subjects as an indicator and was based on a sandy feeling in oral cavity or a taste thereof during disintegrating. The feeling in oral cavity was evaluated as three levels, i.e. "Good" means that a feeling in oral cavity is good, "Not bad" means that a feeling in oral cavity is not bad, and "Bad" means that a feeling in oral cavity is bad. The result was shown in Table 3.

**Table 3**

| | Good | Not bad | Bad |
|---|---|---|---|
| Example 1 | 9 | 1 | 0 |
| Example 2 | 8 | 2 | 0 |
| Reference Example 3 | 0 | 0 | 10 |

As shown in the above Table 3, the powders of Examples 1 and 2 were agreeable and the feelings in oral cavity were good. On the other hand, with respect to the powder of Reference Example 3, the subjects felt sandy in oral cavity and the feelings in oral cavity were bad. So the powder was thought to be hard to be swallowed.

### INDUSTRIAL APPLICABILITY

As mentioned hereinbefore, the cilostazol powder of the present invention has orally disintegrating properties which has never been known in a powder formulation, and can be taken without water and be disintegrated in oral cavity, which is convenient to a lot of patients to whom cilostazol is applied, especially aged patients and patients suffering from dysphagia, and hence, the present powder can widely be used in the pharmaceutical field.

## Claims

1. An orally disintegrating powder comprising cilostazol as an active ingredient and mannitol in an amount of 70 % by weight or more.

2. The powder according to claim 1, wherein an amount of the cilostazol is 10 % by weight to 30 % by weight.

3. The powder according to claim 1 or 2, wherein a single dosage unit of cilostazol is 50 mg to 100 mg.

4. The powder according to any one of claims 1 to 3, wherein the mannitol is D-mannitol.

5. The powder according to any one of claims 1 to 4, wherein the mannitol is derived from corn starch.

6. The powder according to any one of claims 1 to 5, wherein the mannitol has a mean particle size of 20 micrometers to 80 micrometers.

7. The powder according to any one of claims 1 to 6, which further comprises 5% by weight or less microcrystalline cellulose.

8. Use of the powder according to any one of claims 1 to 7 for preparing a medicament for preventing relapse of cerebral infarction.

## Patentansprüche

1. Im Mund zerfallendes Pulver, das Cilostazol als Wirkstoff und Mannit in einer Menge von 70 Gew.-% oder mehr umfasst.

2. Pulver gemäß Anspruch 1, wobei die Menge des Cilostazols 10 Gew.-% bis 30 Gew.-% beträgt.

3. Pulver gemäß Anspruch 1 oder 2, wobei eine Einzeldosiseinheit von Cilostazol 50 mg bis 100 mg beträgt.

4. Pulver gemäß einem der Ansprüche 1 bis 3, wobei es sich bei dem Mannit um D-Mannit handelt.

5. Pulver gemäß einem der Ansprüche 1 bis 4, wobei das Mannit von Maisstärke abgeleitet ist.

6. Pulver gemäß einem der Ansprüche 1 bis 5, wobei das Mannit eine mittlere Teilchengröße von 20 Mikrometer bis 80 Mikrometer hat.

7. Pulver gemäß einem der Ansprüche 1 bis 6, das weiterhin 5 Gew.-% oder weniger mikrokristalline Cellulose umfasst.

8. Verwendung des Pulvers gemäß einem der Ansprüche 1 bis 7 zur Herstellung eines Medikaments zur Prävention eines Rückfalls nach einem Hirninfarkt.

## Revendications

1. Poudre à délitement oral comprenant du cilostazol en tant que principe actif et du mannitol dans une quantité de 70 % en poids ou plus.

2. Poudre selon la revendication 1, où une quantité du cilostazol est de 10 % en poids à 30 % en poids.

3. Poudre selon la revendication 1 ou 2, où une seule unité posologique de cilostazol est de 50 mg à 100 mg.

4. Poudre selon l'une quelconque des revendications 1 à 3, où le mannitol est du D-mannitol.

5. Poudre selon l'une quelconque des revendications 1 à 4, où le mannitol est dérivé d'amidon de maïs.

6. Poudre selon l'une quelconque des revendications 1 à 5, où le mannitol a une taille de particule moyenne de 20 micromètres à 80 micromètres.

7. Poudre selon l'une quelconque des revendications 1 à 6, qui comprend en outre 5 % en poids ou moins de cellulose microcristalline.

8. Utilisation de la poudre selon l'une quelconque des revendications 1 à 7, pour préparer un médicament destiné à prévenir une rechute d'infarctus cérébral.
